# EUROPÄISCHE PATENTSCHRIFT

(11) **EP 1 463 552 B1**
(45) Veröffentlichungstag und Bekanntmachung des Hinweises auf die Patenterteilung: **10.03.2010**
(21) Anmeldenummer: 03702172.2
(22) Anmeldetag: 08.01.2003
(51) Int. Cl.: A61M 5/32, A61M 5/28, A61M 5/34

(54) **INJEKTIONSSPRITZKOPF MIT ORIGINALITÄTSVERSCHLUSS**
HYPODERMIC-SYRINGE HEAD COMPRISING A TAMPER-PROOF SEAL
TETE DE SERINGUE A FERMETURE DE SECURITE

(30) Priorität: 09.01.2002 AT 262002
(43) Veröffentlichungstag der Anmeldung: 06.10.2004
(73) Patentinhaber: PICKHARD, Brigitte, 2203 Grossebersdorf (AT)
(72) Erfinder: PICKHARD, Brigitte, 2203 Grossebersdorf (AT)
(74) Vertreter: Holzer, Walter
(86) Internationale Anmeldenummer: PCT/AT2003/000004
(87) Internationale Veröffentlichungsnummer: WO 2003/057289

(56) Entgegenhaltungen:
- WO-A-88/00478
- WO-A-96/03171
- WO-A-97/49444
- US-A- 5 078 698

## Beschreibung

Die Erfindung betrifft einen Injektionsspritzenkopf mit Originalitätsverschluß für eine Spritzenzylinder-Kolbeneinheit, die einen Zylinderhals aufweist, der mit einem axialen Flüssigkeitsaustrittskanal und einem Umfangswulst zur Verankerung des Injektionsspritzenkopfes versehen ist, mit einer Injektionskanüle, die in einem Kanülenhalter montiert ist, einer Kanülenhalterführungshülse, welche den Kanülenhalter aufnimmt, und einer Schutzkappe, an die über eine Sol-lbruchstelle ein die Kanülenhalterführungshülse aufnehmender, mit dem Umfangswulst des Zylinderhalses zusammenwirkender Verankerungsteil anschließt, wobei die Schutzkappe am Innenumfang in Axialrichtung verlaufende Rippen aufweist, welche in entsprechende Nuten der Kanülenhalterführungshülse für einen Drehantrieb derselben in einem vorbestimmten Drehsinn eingreifen, und wobei im Verankerungsteil zwischen Kanülenhalterführungshülse und Zylinderhals eine vom distalen Ende der Injektionskanüle durchstoßbare Dichtungsscheibe aufgenommen ist.

In der vorliegenden Beschreibung werden die Richtungen "proximal" und "distal" von der Seite des Patienten her betrachtet definiert.

Ein derartiger Injektionsspritzenkopf ist zur Herstellung sogenannter Fertigspritzen bestimmt, d.h. von Spritzen, die mit einem flüssigen Arzneimittel gefüllt und einer Injektionskanüle versehen steril abgepackt und gebrauchsfertig angeboten werden. Es ist wesentlich, daß diese Fertigspritzen mit einem Originalitätsverschluß versehen sind, der es gestattet zu erkennen, ob die Spritze noch mit der Originalfüllung gefüllt und steril ist. Der Injektionsspritzenkopf sollte die Kanüle ausreichend schützen, so daß es bei der Endmontage des Spritzenkopfes an der Spritzenzylinder-Kolbeneinheit zu keiner Beschädigung oder Verunreinigung der Kanüle kommen kann. Ferner ist für die Massenfertigung ein einfacher Aufbau der Injektionsspritze erforderlich.

Bei Injektionsspritzenköpfen ergibt sich in der Praxis ferner das Problem, daß bei einem unbeabsichtigten Verdrehen der Schutzkappe gegenüber dem Verankerungsteil in einem falschen Drehsinn zwar die Sollbruchstelle durchbrochen wird, die Kanüle jedoch nicht durch die Dichtungsscheibe hindurch und in Verbindung mit dem Spritzenzylinder getrieben wird. Ein Öffnen in der falschen Richtung macht daher die Injektionsspritzenköpfe der bekannten Art wertlos. Es weist deshalb bei einem Injektionsspritzenkopf der einleitend angegebenen Art (AT-400 926) die Schutzkappe am Innenumfang im Bereich der Kanülenhalterführungshülse eine Anschlagrippe auf, die in der Vorgebrauchstellung der Schutzkappe an einer Seite eines am Außenumfang der Kanülenhalterführungshülse vorspringenden Anschlages anliegt. Die Aktivierung der Spritze erfolgt durch Verdrehen der Schutzkappe, wodurch die Sollbruchstelle reißt und der Kanülenhalter in die Kanülenhalterführungshülse eingeschraubt wird. Dabei durchstößt das distale Ende der Injektionskanüle drehend die Dichtungsscheibe. Die Schutzkappe wird dann in axialer Richtung abgezogen und die Spritze ist zur Injektion bereit.

Der Anschlag am Außenumfang der Kanülenhalterführungshülse in Verbindung mit der Anschlagrippe in der Schutzkappe soll gewährleisten, daß die Schutzkappe aus ihrer Vorgebrauchstellung, d.h. bei noch geschlossener Sollbruchstelle, nur in jener Richtung gedreht werden kann, welche ein Einschrauben des Kanülenhalters in die Kanülenhalterführungshülse bewirkt. Es sollte also eine versehentliche Verdrehung der Schutzkappe in der anderen Richtung, wobei die Sollbruchstelle aufgetrennt würde, ohne die Injektionskanüle in Wirkstellung zu bringen, ausgeschlossen sein. In der Praxis zeigt sich jedoch, daß der aus konstruktiven Gründen relativ kleine Anschlag insbesondere bei aus weichem Material bestehenden Schutzkappen nicht wirksam ist, so daß es zu einer Zerstörung der Originalitätssicherung kommen kann und der Kanülenhalter aus der Kanülenhalterführungshülse herausbewegt wird. Die Spritze ist dann aber unbrauchbar. Außerdem hat sich in der Praxis gezeigt, daß bei dem bekannten Spritzenkopf beim Aktivieren und Durchstechen der Gummidichtungsscheibe durch die Drehbewegung der Kanülenspritze Scheibenpartikel mitgerissen werden, welche das Produkt für eine pharmazeutische Anwendung unbrauchbar machen.

Ein Injektionsspritzenkopf der einleitend angegebenen Art ist auch aus der WO 97 49 444 bekannt. Ein V-förmiger Führungskanal am Kanülenträger, an dessen Spitze der Führungszapfen der Kanülenhalterführungshülse eingesetzt ist, ermöglicht es, die Kanülenhalterführungshülse durch Drehung der Schutzkappe in beiden Drehrichtungen zur Dichtscheibe zu bewegen. Das Erfordernis eines sicheren Drehanschlages für eine Drehrichtung entfällt also.

Ein Nachteil dieser Konstruktion besteht darin, daß die Zusammenführung von Kanüle und Spritzenzylinder auch durch Drehung der Schutzkappe entgegen dem Uhrzeigersinn erfolgen kann, was der Intuition vieler Anwender entgegensteht. Es kann daher vorkommen, daß Anwender spontan zunächst eine Linksdrehung beginnen oder vollenden, anschließend den Rechtsdrehsinn von Schrauben, Schraubverschlüssen und anderem erinnern und aus einem Sicherheitswunsch heraus eine anschließende Rechtsdrehung vollziehen. Dies bewirkt ein zweimaliges Durchstechen der Dichtscheibe durch das distale Ende der Kanüle, was einen ausreichend dichten Abschluß zwischen Kanülenaußenumfang und durchstochener Dichtscheibe verhindert. Bei Betätigung der Spritze kann es zu ungewolltem Austritt des Medikamentes an dieser Stelle kommen.

Ein Injektionsspritzenkopf der einleitend angegebenen Art mit einer 1-Richtung-Drehsicherung offenbart die WO 96 03 171. Die Drehbewegung der Schutzkappe wird hier in eine Schraubbewegung der Einheit aus Kanüle und Kanülenhalter umgesetzt. Dadurch dreht sich das distale Ende der Kanüle in die Membran der Dichtscheibe. Zum einen erfordert dies, daß das distale Ende der Kanüle nicht angeschrägt, sondern axial spitz zulaufend und mit einer seitlichen Öffnungsbohrung für den Eintritt des flüssigen Medikamentes versehen ist, was ein umfangreicheres Herstellungsverfahren der Kanüle erzwingt. Zum anderen kommt es bei Zusammenfügung von Kanüle und Spritzenzylinder zu Reibungen zwischen Kanülenaußenwand bzw. seitlicher Öffnungsbohrung am distalen Kanülenende und der durchstochenen Membran der Dichtscheibe, was den dichten Abschluß zwischen Kanüle und Membran gefährden kann.

Die vorliegende Erfindung zielt darauf ab, diese Nachteile zu vermeiden und einen geraden Durchstoß der Dichtscheibe durch die Kanüle mittels einer Drehung der Schutzkappe im Uhrzeigersinn herbeizuführen. Zudem soll neben der Drehrichtung im Uhrzeigersinn keine zweite Drehrichtung an der Schutzkappe auszuführen sein. Die Erfindung erreicht dies dadurch, daß die aus Hartkunststoff bestehende Kanülenhalterführungshülse einen ebenfalls aus Hartkunststoff bestehenden Antriebsteil aufnimmt, der über Antriebsrippen mit den Rippen der Schutzkappe in Eingriff steht und, wie an sich bekannt, mit einer im wesentlichen wendelförmigen Führungsnut versehen ist, die mit einem Führungselement der Kanülenhalterführungshülse in Eingriff steht, daß die Führungsnut an ihrem unteren Ende in eine axial verlaufende, am unteren Ende des Antriebsteiles ausmündende Einlaufnut übergeht, welche eine Anschlagkante gegen Drehung entgegen dem vorbestimmten Drehsinn bildet, daß der Kanülenhalter in der Kanülenhalterführungshülse axial gleitverschieblich gelagert ist und daß die Dichtungsscheibe an ihrer distalen Unterseite mit einer ringförmigen Dichtungslippe versehen ist.

Eine bevorzugte Ausführungsform der Erfindung zeichnet sich dadurch aus, daß die Kanülenhalterführungshülse in ihrem oberen Innenabschnitt mit axial verlaufenden Ausnehmungen zur Aufnahme von Führungsnasen des Kanülenhalters versehen ist.

Bedingt durch das Führungselement der Führungshülse, das mit der Führungsnut des Antriebsteiles leicht bewegbar im Eingriff steht, wobei beide Teile aus Hartkunststoff hergestellt sind, wird einerseits eine ideale Kulisse für die Drehbewegung des Antriebsteiles in der Führungshülse geschaffen, anderseits wird durch die untere Einlaufnut mit der Anschlagkante, an welcher das Führungselement in der Vorgebrauchstellung ansteht, ein sicherer Anschlag geschaffen, so daß auch bei falscher Handhabung (d.h. einer Drehung gegen den Uhrzeigersinn) das Kanülensystem nicht aktiviert wird. Mit anderen Worten aktiviert eine versehentliche falsche Drehung der Schutzkappe gegen die Pfeilrichtung die Spritze nicht, sondern es wird lediglich die Originalitätssicherung (Sollbruchstelle) zerstört, während dann bei einer neuerlichen Drehung der aufgedrehten Schutzkappe in der richtigen Drehrichtung (im Uhrzeigersinn) das Kanülensystem problemlos aktiviert wird und das Medikament ausgespritzt werden kann.

Durch eine steile Ausbildung der Führungsnut (Kulisse) im Antriebsteil kann erreicht werden, daß ein Durchdringen der zentralen Membrane der Dichtungsscheibe bereits nach einer 1/4 Umdrehung gewährleistet ist. Der wesentliche und entscheidende Vorteil des Kanülensystems gegenüber den anderen bekannten Systemen mit geschlossener Dichtungsscheibe (die zur sicheren Trennung zwischen Medikament und Kanülensystem unbedingt erforderlich ist) besteht im Vermeiden einer Drehbewegung der Kanüle zum Durchstechen der geschlossenen Gummimembrane und die Anwendung einer reinen Schiebebewegung. Durch den Einsatz einer geschlossenen Dichtungsscheibe aus Pharmagummi, die zusätzlich an ihrer distalen Seite mit einer ringförmigen, dünnen und elastischen Dichtlippe versehen ist, wird sichergestellt, daß während der möglicherweise bis zu 5 Jahren dauernden Lagerzeit der Spritze.das Medikament nicht in den aufgesetzten Spritzenkopf gelangen kann, wodurch ein Auskristallisieren des Medikamentes in diesem Bereich bzw. sogar ein mögliches Wachstum von Keimen im Injektionsspritzenkopf verhindert wird.

Die Erfindung ermöglicht zugleich eine einfache Montage und Positionierung des Antriebsteiles in der Kanülenhalterführungshülse, weil der Antriebsteil über die Einlaufnut einfach eingeschoben und positioniert werden kann. Dann wird die Kanülenhalterführungshülse mit den vier Rippen in die entsprechenden Ausnehmungen der Schutzkappe gesteckt und gleichzeitig positioniert, wobei die vier Rippen außerdem ein Verdrehen der Führungshülse in der Schutzkappe verhindern.

Die Erfindung wird nachstehend an Hand eines in den Zeichnungen dargestellten Ausführungsbeispieles näher erläutert. Es zeigen:
Fig. 1 einen erfindungsgemäßen Injektionsspritzenkopf in gesprengter Darstellung in Verbindung mit einer nur teilweise dargestellten Spritzenzylinder-Kolbeneinheit,
die Fig. 2a und 2b den Injektionsspritzenkopf nach Fig. 1 in zusammengebautem Zustand im Axialschnitt;
die Fig. 3 bis 5 Querschnitte nach den Linien III-III bzw. IV-IV bzw. V-V in Fig. 2;
Fig. 6 einen Axialschnitt durch die Kanülenhalterführungshülse mit Antriebsteil;
Fig. 7 eine Perspektivansicht des Antriebsteiles;
Fig. 8 eine Perspektivansicht der Führungshülse; und
Fig. 9 eine Untersicht der Führungshülse.

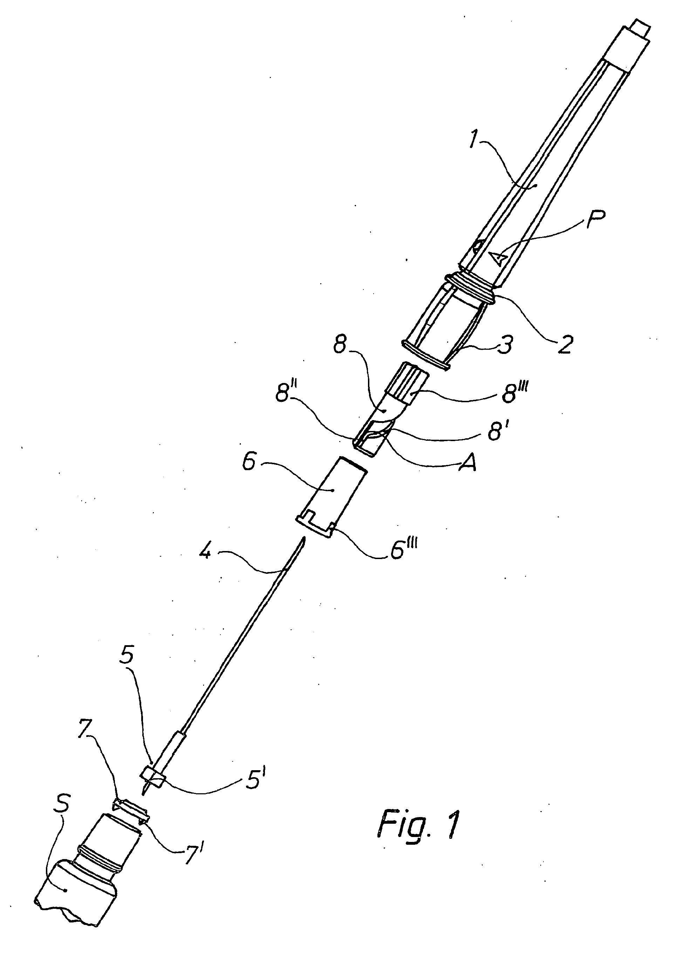

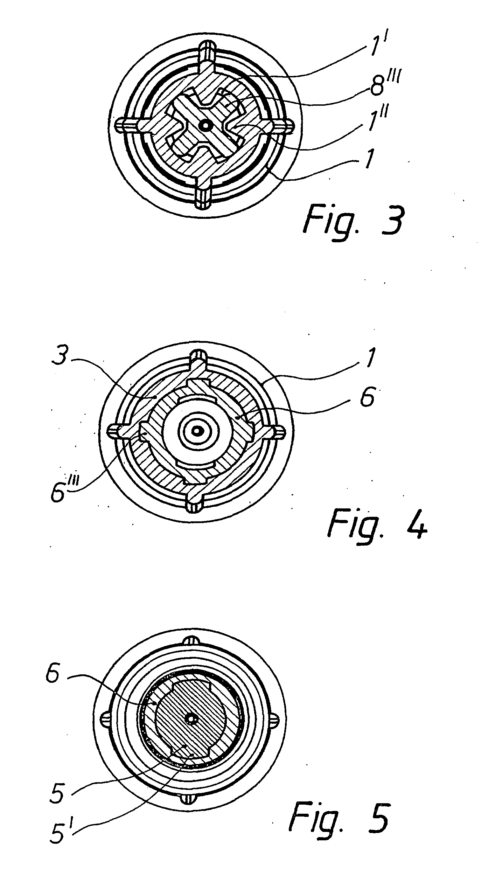

Der Injektionsspritzenkopf setzt sich gemäß Fig. 1 im wesentlichen aus fünf'Montageteilen zusammen, u. zw. einer Schutzkappe 1, die über eine Sollbruchstelle 2 einstückig mit einem am Spritzenzylinder S befestigbaren Verankerungsteil 3 verbunden ist, einer Injektionskanüle 4, die in einem Kanülenhalter 5 fest verankert ist, z.B. durch Hochfrequenzverschweißen oder Verkleben, einer Kanülenhalterführungshülse 6 und einer Dichtungsscheibe 7. Gemäß Fig. 1 ist ferner ein Antriebsteil 8 vorgesehen, der in die Kanülenhalterführungshülse 6 geschoben wird, worauf diese nach dem Einbau des Kanülenhalters 5 in die, Schutzkappe 1 gesteckt wird. Der Antriebsteil 8 hat eine steile, z.B. unter 45° verlaufende wendelförmige Führungsnut 8' mit einer axialen unteren Einlaufnut 8". An seinem oberen Ende bildet der Antriebsteil 8 durch einen im wesentlichen kreuzförmigen Querschnitt vier Rippen 8"', die gemäß Fig. 3 in Ausnehmungen 1' der Schutzkappe 1 ragen, die Zwischenräume zwischen keilförmigen Rippen 1" der Kappe 1 bilden.

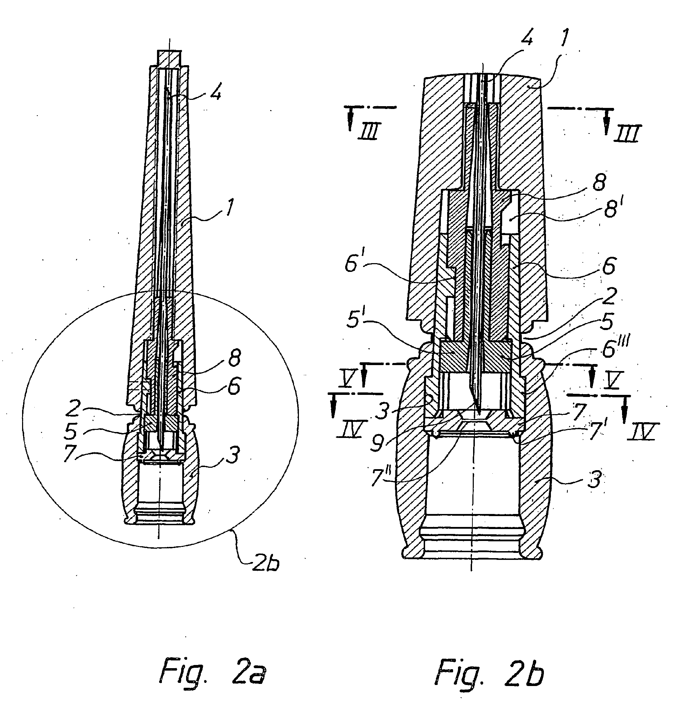

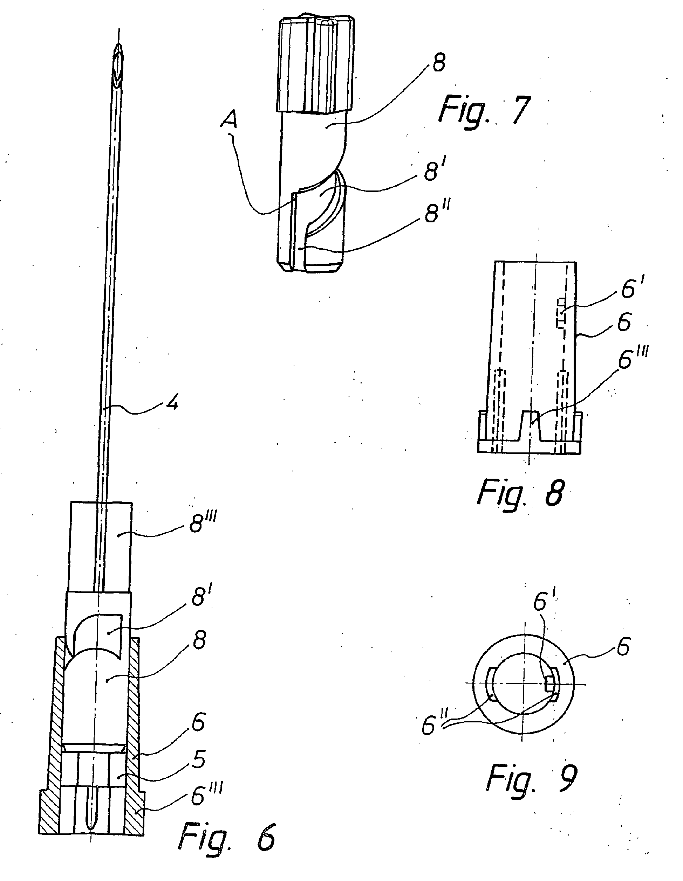

Beim Zusammenbau des Injektionsspritzenkopfes wird der Kanülenhalter 5 über Nasen 5' in axiale Ausnehmungen 6" der Kanülenhalterführungshülse 6 auf Anschlag eingesteckt (Fig. 6), so daß das distale Ende 9 der Kanüle 4 unmittelbar vor der zentralen Membrane der Dichtungsscheibe 7 steht (Fig. 2a, b). Die Kanülenhalterführungshülse 6 (Fig. 8, 9) wird anschließend in den Verankerungsteil 3 der Schutzkappe 1 von deren distalem Ende her eingepreßt (Fig. 1), wobei durch eine konische Ausbildung des Außenumfanges der Kanülenhalterführungshülse 6 und eine korrespondierende konische Ausbildung der Innenwand des Verankerungsteiles 3 ein einwandfreier Sitz erzielt wird. Zur Sicherung gegen Verdrehen sind an der Kanülenhalterführungshülse 6 Vorsprünge 6"' vorgesehen, die in entsprechende Ausnehmungen 3' des Verankerungsteiles 3 passen. Zum Abschluß wird die Dichtungsscheibe 7 in eine Hinterschneidung des Verankerungsteiles 3 eingesetzt. Die in den Fig. 1 und 2a, b dargestellte Dichtungsscheibe 7 bildet in ihrem zentralen Bereich eine Membrane 7". Die Dichtungsscheibe 7 hat ferner an ihrer distalen Unterseite eine umlaufende Dichtlippe 7', die zum Ausgleich von Höhentoleranzen und zur besseren Abdichtung zwischen dem Spritzenzylinder und dem Spritzenkopf dient.

Der auf diese Weise fertiggestellte Injektionsspritzenkopf ist in den Fig. 2a, b gezeigt und kann transportiert, sterilisiert und zwischengelagert werden, ohne daß die Gefahr einer Verunreinigung oder Beschädigung der Injektionskanüle 4 besteht.

Beim Aktivieren des Kanülensystems durch Abdrehen der Schutzkappe 1 in Pfeilrichtung P, bei gleichzeitiger Zerstörung der Originalitätssicherung, d.h. der Sollbruchstelle 2, wird die Drehbewegung der Schutzkappe 1, in welche die Kanülenhalterführungshülse 6 gesichert sitzt, über die vier Antriebsrippen 1" und 8"' und über die Führungsnut 8' (Kulisse) des Antriebsteiles 8 auf den Kanülenhalter 5 übertragen, der mit seinen beiden Führungsnasen 5' in Ausnehmungen 6" der Führungshülse 6 gegen Verdrehung gesichert sitzt und im Antriebsteil 8 sicher gehalten ist und nunmehr zwangsläufig axial ohne Drehbewegung mit der Kanülenspitze in Richtung der Dichtungsscheibe 7 und durch deren zentrale Membrane 7" geschoben wird. Die axiale Kante der Einlaufnut 8" bildet einen Anschlag A gegen Drehung in der falschen Richtung.

Durch das Umwandeln der Drehbewegung der Schutzkappe 1 in eine Schiebebewegung des Kanülenhalters 5 kann es beim Durchstechen der Membrane der Dichtungsscheibe durch die Kanülenspitze zu keiner (Gummi-)Partikelbildung mehr kommen, weil eine partikelverursachende (schneidende bzw. schabende) Drehbewegung eliminiert ist.

Die Erfindung kann im Rahmen des allgemeinen Erfindungsgedankens verschiedentlich abgewandelt werden.

## Patentansprüche

1. Injektionsspritzenkopf mit Originalitätsverschluß für eine Spritzenzylinder-Kolbeneinheit, die einen Zylinderhals aufweist, der mit einem axialen Flüssigkeitsaustrittskanal und einem Umfangswulst zur Verankerung des Injektionsspritzenkopfes versehen ist, mit einer Injektionskanüle, die in einem Kanülenhalter montiert ist, einer Kanülenhalterführungshülse, welche den Kanülenhalter aufnimmt, und einer Schutzkappe, an die über eine Sollbruchstelle ein die Kanülenhalterführungshülse aufnehmender, mit dem Umfangswulst des Zylinderhalses zusammenwirkender Verankerungsteil anschließt, wobei die Schutzkappe am Innenumfang in Axialrichtung verlaufende Rippen aufweist, welche in entsprechende Nuten der Kanülenhalterführungshülse für einen Drehantrieb derselben in einem vorbestimmten Drehsinn eingreifen, wobei im Verankerungsteil zwischen Kanülenhalterführungshülse und Zylinderhals eine, vom distalen Ende der Injektionskanüle durchstoßbare Dichtungsscheibe aufgenommen ist, wobei die Kanülenhalterführungshülse einen Antriebsteil aufnimmt der über Antriebsrippen (8"') mit den Rippen (1") der Schutzkappe (1) in Eingriff steht und, wie an sich bekannt, mit einer im wesentlichen wendelförmigen Führungsnut (8') versehen ist, die mit einem Führungselement (6') der Kanülenhalterführungshülse (6) in Eingriff steht, wobei die Führungsnut (8') an ihrem unteren Ende in eine axial verlaufende, am unteren Ende des Antriebsteiles ausmündende Einlaufnut (8") übergeht und wobei der Kanülenhalter (5) in der Kanülenhalterführungshülse (6) axial gleitverschieblich gelagert ist, **dadurch gekennzeichnet, daß** die aus Hartkunststoff bestehende Kanülenhalterführungshülse (6) den ebenfalls aus Hartkunststoff bestehenden Antriebsteil (8) aufnimmt daß die Einlaufnut (8") eine Anschlagkante (A) gegen Drehung entgegen dem vorbestimmten Drehsinn bildet, und daß die Dichtungsscheibe (7) an ihrer distalen Unterseite mit einer ringförmigen Dichtungslippe (7') versehen ist.

2. Injektionsspritzenkopf nach Anspruch 1, **dadurch gekennzeichnet, daß** die Kanülenhalterführungshülse (6) in ihrem unteren Innenabschnitt mit axial verlaufenden Ausnehmungen (6") zur Aufnahme von Führungsnasen (5') des Kanülenhalters (5) versehen ist.

## Claims

1. Hypodermic-syringe head comprising a tamper-proof seal for a syringe-cylinder plunger unit having a cylinder neck which is provided with an axial liquid outlet channel and a peripheral collar for retaining the hypodermic-syringe head, comprising a hypodermic needle which is mounted in a needle holder, a needle-holder guide sleeve which receives the needle holder, and comprising a protective cap to which a retaining portion is connected via an intended break-off point, said retaining portion receiving the needle-holder guide sleeve and cooperating with the peripheral collar of the cylinder neck, the protective cap comprising ribs extending in an axial direction at the inner periphery, said ribs engaging in corresponding grooves in the needle-holder guide sleeve to create a rotatory drive in a predetermined direction of rotation, a sealing ring which may be pierced by the distal end of the hypodermic needle being received in the retaining portion between the needle-holder guide sleeve and the cylinder neck, the needle-holder guide sleeve receiving a drive portion which is engaged with the ribs (1") in the protective cap (1) via driving ribs (8"') and, as is known per se, is provided with a substantially helical guide groove (8') engaged with a guide member (6') of the needle-holder guide sleeve (6), the guide groove (8') merging at its lower end into an axially extending inlet groove (8") opening out at the lower end of the drive portion, and the needle holder (5) being axially slidingly mounted in the needle-holder guide sleeve (6), **characterised in that** the needle-holder guide sleeve (6) made of hard plastics material receives the drive portion (8), which is also made of hard plastics material, **in that** the inlet groove (8") forms a stop edge (A) preventing rotation counter to the predetermined direction of rotation, and **in that** the sealing ring (7) is provided with an annular sealing lip (7') on its distal underside.

2. Hypodermic-syringe head according to claim 1, **characterised in that** the needle-holder guide sleeve (6) is provided with axially extending recesses (6") in its lower inner portion for receiving guide lugs (5') of the needle holder (5).

## Revendications

1. Tête de seringue pour injection, comportant une fermeture originale, pour une unité de cylindre de seringue-piston, qui présente un col cylindrique qui est doté d'un canal axial de sortie de liquide et d'un bourrelet périphérique pour l'ancrage de la tête de seringue, une canule d'injection qui est montée dans un support de canule, une gaine de guidage du support de canule qui reçoit le support de canule et un capuchon de protection auquel est adjointe, par une position de rupture théorique, une pièce d'ancrage coopérant avec le bourrelet périphérique du col de cylindre, le capuchon de protection présentant sur le pourtour intérieur des nervures évoluant en direction axiale qui sont en prise dans les rainures correspondantes de la gaine de guidage du support de canule, pour un mouvement de rotation de celle-ci dans un sens de rotation prédéterminé, dans la pièce d'ancrage entre la gaine de guidage du support de canule et le col cylindrique, un disque d'étanchéité étant reçu de l'extrémité distale de la canule d'injection pouvant être poussée, la gaine de guidage du support de canule recevant une partie d'entraînement qui est en prise avec les nervures (1") du capuchon de protection (1) par le biais des nervures d'entraînement (8"'), et est dotée de façon connue d'une rainure de guidage (8') de forme essentiellement spiralée qui est en prise avec un élément de guidage (6') de la gaine de guidage du support de canule (6), la rainure de guidage (8') évoluant au niveau de son extrémité inférieure en une rainure d'admission axiale, débouchant au niveau de l'extrémité inférieure de la pièce d'entraînement et le support de canule (5) étant placé dans la gaine de guidage de support de canule (6) de façon à pouvoir coulisser axialement, **caractérisé en ce que** la gaine de guidage du support de canule consistant en matière plastique dure (6) reçoit la partie d'entraînement (8) constituée également de matière plastique dure, **en ce que** la rainure d'admission (8") forme un bord de butée (A) contre une rotation contraire au sens de rotation prédéterminé, et que le disque d'étanchéité (7) est doté au niveau de sa face inférieure distale, d'une lèvre d'étanchéité circulaire (7').

2. Tête de seringue selon la revendication 1, **caractérisée en ce que** la gaine de guidage de support de canule (6) est dotée dans la section interne inférieure, d'évidements évoluant axialement (6") pour recevoir des proéminences de guidage (5') du support de canule (5).
